(19) **Europäisches Patentamt / European Patent Office / Office européen des brevets**

(11) **EP 2 799 131 A1**

(12) **DEMANDE DE BREVET EUROPEEN**

(43) Date de publication:
05.11.2014 Bulletin 2014/45

(51) Int Cl.:
*B01J 23/883* (2006.01)          *B01J 23/28* (2006.01)
*B01J 37/02* (2006.01)          *B01J 37/20* (2006.01)
*C10G 45/08* (2006.01)          *C10G 47/12* (2006.01)
*B01J 31/22* (2006.01)          *B01J 31/02* (2006.01)

(21) Numéro de dépôt: 14305437.7

(22) Date de dépôt: 26.03.2014

(84) Etats contractants désignés:
AL AT BE BG CH CY CZ DE DK EE ES FI FR GB
GR HR HU IE IS IT LI LT LU LV MC MK MT NL NO
PL PT RO RS SE SI SK SM TR
Etats d'extension désignés:
BA ME

(30) Priorité: 30.04.2013 FR 1353940

(71) Demandeur: IFP Energies nouvelles
92852 Rueil-Malmaison Cedex (FR)

(72) Inventeurs:
• Alphazan, Thibault
69780 Mions (FR)
• Bonduelle-Skrzypczak, Audrey
69340 Francheville (FR)
• Legens, Christele
69002 Lyon (FR)
• Raybaud, Pascal
69003 Lyon (FR)
• Coperet, Christophe
CH-8093 Zürich (CH)

(54) **Procédé de preparation d'un catalyseur a base de molybdene utilisable en hydrotraitement ou en hydrocraquage**

(57) L'invention concerne un procédé de préparation de catalyseur à base de molybdène destiné aux procédés d'hydrotraitement ou d'hydrocraquage.

L'invention concerne un procédé de préparation d'un catalyseur permettant des réactions d'hydrogénation dans les procédés d'hydrotraitement et d'hydrocraquage. Ledit catalyseur est préparé à partir d'au moins un précurseur mononucléaire à base de molybdène (Mo), sous sa forme monomérique ou dimérique, présentant au moins une liaison Mo=O ou Mo-OR ou au moins une liaison Mo=S ou Mo-SR où [R = $C_xH_y$ où $x \geq 1$ et $(x-1) \leq y \leq (2x+1)$ ou R = $Si(OR')_3$ ou R = $Si(R')_3$ où R' = $C_{x'}H_{y'}$ où $x' \geq 1$ et $(x'-1) \leq y' \leq (2x'+1)$], et à partir éventuellement d'au moins un élément promoteur du groupe VIII. Lesdits précurseurs sont déposés sur un support oxyde adapté au procédé dans lequel il est utilisé, ledit catalyseur étant séché à une température inférieure à 200°C, puis avantageusement sulfuré avant d'être mis en oeuvre dans ledit procédé.

EP 2 799 131 A1

**Description**

La présente invention décrit un procédé de préparation d'un catalyseur à base de molybdène (Mo) particulièrement efficace dans les réactions d'hydrogénation intervenant dans les procédés d'hydrotraitement et d'hydrocraquage, ainsi que dans les réactions d'hydrocraquage à proprement parler intervenant dans les procédés d'hydrocraquage.

La présente invention a également pour objet l'utilisation dudit catalyseur dans les procédés d'hydrotraitement et/ou d'hydrocraquage.

État de la technique antérieure

Généralités sur les catalyseurs d'hydrotraitement (HDT) et d'hydrocraquage (HCK) de charges hydrocarbonées.

La composition et l'utilisation des catalyseurs d'hydrotraitement et d'hydrocraquage de charges hydrocarbonées sont respectivement bien décrites dans les ouvrages : "Catalysis by transition metal sulphides, From Molecular Theory to Industrial Application", 2013, H. Toulhouat, P. Raybaud et "Hydrocracking Science and Technology", 1996, J. Scherzer, A. J. Gruia, Marcel Dekker Inc.

Ainsi, les catalyseurs utilisés dans les procédés de raffinage, qu'ils soient destinés aux réactions d'hydrotraitement ou d'hydrocraquage, se caractérisent généralement par une fonction hydro-déshydrogénante apportée par la présence d'une phase active à base d'au moins un métal du groupe VIB et éventuellement au moins un métal du groupe VIII du tableau périodique des éléments. Les formulations les plus courantes sont de type cobalt-molybdène (CoMo), nickel-molybdène (NiMo) et nickel-tungstène (NiW). Ces catalyseurs peuvent se présenter sous forme massique (valable spécifiquement pour les catalyseurs d'hydrotraitement) ou bien à l'état supporté mettant alors en jeu un solide poreux de nature différente. Dans ce dernier cas, le support poreux est généralement un oxyde amorphe ou mal cristallisé (alumine, aluminosilicate, etc.), éventuellement associé à un matériau zéolithique ou non. Après préparation, au moins un métal du groupe VIB et éventuellement au moins un métal du groupe VIII constitutif(s) desdits catalyseurs se présente(nt) souvent sous une forme oxydée. La forme active et stable pour les procédés d'hydrocraquage (HCK) et d'hydrotraitement (HDT) étant la forme sulfurée, ces catalyseurs doivent subir une étape de sulfuration. Celle-ci peut être réalisée dans l'unité du procédé associé (on parle alors de sulfuration in-situ) ou préalablement au chargement du catalyseur dans l'unité (on parle alors de sulfuration ex-situ).

Il est généralement connu de l'Homme du métier que de bonnes performances catalytiques dans les domaines d'application mentionnés ci-dessus sont fonction : 1) de la nature de la charge hydrocarbonée à traiter, 2) du procédé employé, 3) des conditions opératoires de fonctionnement choisies et 4) du catalyseur utilisé. Dans ce dernier cas, il est également admis qu'un catalyseur présentant un fort potentiel catalytique se caractérise : 1) par une fonction hydro-déshydrogénante optimisée (phase active associée parfaitement dispersée à la surface du support et présentant une teneur en phase active élevée) et 2) dans le cas particulier des procédés mettant en jeu de réactions d'HCK, par un bon équilibre entre ladite fonction hydro-déshydrogénante et la fonction craquante. Notons également que, idéalement, quelle que soit la nature de la charge hydrocarbonée à traiter, le catalyseur doit pouvoir présenter une accessibilité des sites actifs vis-à-vis des réactifs et produits de réactions tout en développant une surface active élevée, ce qui peut conduire à des contraintes spécifiques en terme de structure et de texture propres au support oxyde constitutif desdits catalyseurs.

Les méthodes usuelles conduisant à la formation de la phase hydro-déshydrogénante des catalyseurs d'hydrotraitement et d'hydrocraquage consistent en un dépôt de précurseur(s) comprenant au moins un métal du groupe VIB et éventuellement au moins un métal du groupe VIII sur un support oxyde par la technique dite "d'imprégnation à sec" suivi des étapes de maturation, de séchage et éventuellement de calcination conduisant à la formation de la forme oxydée du(des)dit(s) métal(aux) employé(s). Vient ensuite l'étape finale de sulfuration génératrice de la phase active hydro-déshydrogénante comme mentionné ci-dessus.

Les performances catalytiques de catalyseurs issus de ces protocoles de synthèse "conventionnels" ont largement été étudiées. En particulier, il a été montré que, pour des teneurs en métal relativement élevées, des phases réfractaires à la sulfuration formées consécutivement à l'étape de calcination (phénomène de frittage) apparaissent (H. Toulhouat, P. Raybaud "Catalysis by transition metal sulphides, From Molecular Theory to Industrial Application", 2013). Par exemple, dans le cas des catalyseurs de type CoMo ou NiMo supportés sur un support de nature aluminique, il s'agit 1) de cristallites de $MoO_3$, NiO, CoO, $CoMoO_4$ ou $Co_3O_4$, de taille suffisante pour être détectés en DRX, et/ou 2) des espèces du type $Al_2(MoO_4)_3$, $CoAl_2O_4$ ou $NiAl_2O_4$. Les trois espèces précitées contenant l'élément aluminium sont bien connues de l'Homme du métier. Elles résultent de l'interaction entre le support aluminique et les sels précurseurs en solution de la phase active hydro-déshydrogénante, ce qui se traduit concrètement par une réaction entre des ions $Al^{3+}$ extraits de la matrice aluminique et lesdits sels pour former des hétéropolyanions d'Anderson de formule $[Al(OH)_6Mo_6O_{18}]^{3-}$, eux-mêmes précurseurs des phases réfractaires à la sulfuration. La présence de l'ensemble de ces espèces conduit à une perte indirecte non négligeable de l'activité catalytique du catalyseur associé car la totalité des éléments appartenant au moins à un métal du groupe VIB et éventuellement au moins un métal du groupe VIII n'est pas utilisée au maximum de son potentiel puisque une partie de ceux-ci est immobilisée dans des espèces peu ou non actives.

Les performances catalytiques des catalyseurs conventionnels décrits ci-dessus pourraient donc être améliorées, notamment en développant de nouvelles méthodes de préparation de ces catalyseurs qui permettraient :

1) d'assurer une bonne dispersion de la phase hydro-déshydrogénante, en particulier pour des teneurs en métal élevées (par exemple par contrôle de la taille des particules à base de métaux de transition, maintien des propriétés de ces particules après traitement thermique avant sulfuration, etc.),

2) de limiter la formation des espèces réfractaires à la sulfuration, par exemple par un meilleur contrôle des interactions entre la phase active hydro-déshydrogénante (et/ou ses précurseurs) et le support poreux employé, ou par l'obtention d'une meilleure synergie entre les métaux de transition constitutifs de la phase active, etc.

3) d'assurer une bonne diffusion des réactifs et des produits des réactions tout en maintenant des surfaces actives développées élevées (optimisation des propriétés chimiques, texturales et structurales du support poreux).

Le couple NiMo est reconnu comme étant l'un des couples de métaux des groupes VIB et VIII optimal pour l'hydrogénation des aromatiques ainsi que pour l'hydrodéazotation, fonctions clés les réactions d'hydrotraitement ou pour l'hydrocraquage. Malgré les fortes teneurs en NiMo déposées, par voie "classique", à l'aide de précurseurs habituels ($H_3PO_4$, $MoO_3$, $Ni(OH)_2$ ou heptamolybdate d'ammonium et nitrate de Nickel) sur le support et malgré les études paramétriques concernant les étapes de préparation, nous ne parvenons pas à 1) contrôler la dispersion et la morphologie des feuillets et 2) optimiser le taux de promotion de la phase active générée sur les supports : ce sont les clefs essentielles pour largement renforcer le pouvoir hydrogénant de la phase active et ainsi réaliser les réactions d'hydrogénation souhaitées dans les procédés d'hydrotraitement et/ou augmenter le rendement en Distillats Moyens dans le procédé d'Hydrocraquage. Un des challenges scientifiques de ces dernières années consiste à optimiser la phase hydrogénante déposée sur des supports variés de catalyseurs destinés à l'hydrotraitement et l'hydrocraquage.

Il apparaît donc intéressant de trouver des moyens de préparation des catalyseurs d'hydrotraitement, permettant d'obtenir de nouveaux catalyseurs à performances améliorées. L'art antérieur montre que les chercheurs se sont tournés vers plusieurs méthodes parmi lesquelles l'emploi de polyoxométallates divers et variés, l'ajout d'éléments dopants, l'ajout de molécules organiques aux propriétés diverses et variées (solvatation, complexation...) ou enfin, mais dans une moindre mesure, car plus difficiles d'utilisation, l'utilisation de précurseurs mononucléaires.

Préparation des catalyseurs d'hydrotraitement et d'hydrocraquage à partir de polyoxométallates (POM)

L'intérêt des polyoxométallates a déjà été mentionné dans l'état de la technique. Par exemple le document US 2,547,380 mentionne l'utilisation bénéfique des sels d'hétéropolyacides de métaux de groupe VIII tels que les sels de cobalt ou de nickel de l'acide phosphomolybdique ou de l'acide silicomolybdique. Dans ce brevet, l'hétéropolyacide contient toujours du phosphore ou du silicium, ce dernier élément étant l'atome central de la structure. Ces composés ont l'inconvénient de conduire à des rapports atomiques (élément du groupe VIII/élément du groupe VI) limités. A titre d'exemple, le phosphomolybdate de cobalt, de formule a un rapport Co/Mo de 0,125.

Le brevet FR 2,749,778 décrit l'intérêt d'hétéropolyanions de formule générale $M_xAB_{12}O_4$, dans laquelle M est le cobalt ou le nickel, A est le phosphore, le silicium ou le bore et B est le molybdène ou le tungstène. x prend la valeur de 2 ou plus si A est le phosphore, de 2,5 ou plus si A est le silicium et de 3 ou plus si A est le bore. Ces structures ont l'intérêt par rapport aux structures divulguées dans le document US 2,547,380 d'atteindre des rapports atomiques (élément du groupe VIII / élément du groupe VI) supérieurs et ainsi conduire à des catalyseurs plus performants. Cette augmentation du rapport est obtenu grâce à la présence d'au moins une partie du molybdène ou du tungstène à une valence inférieure à sa valeur normale de six telle qu'elle résulte de la composition, par exemple, de l'acide phosphomolybdique, phosphotungstique, silicomolybdique ou silicotungstique.

Le brevet FR 2,764,211 décrit la synthèse et l'utilisation d'hétéropolyanions de formule $M_xAB_{11}O_{40}M'C_{(Z-2x)}$ dans laquelle M est le cobalt ou le nickel, A est le phosphore, le silicium ou le bore et B est le molybdène ou le tungstène, M' est le cobalt, le fer, le nickel, le cuivre ou le zinc, et C est un ion $H^+$ ou un cation alkylammonium, x prend la valeur de O à 4,5, z une valeur entre 7 et 9. Ainsi, cette formule correspond à celle revendiquée dans l'invention FR 2,749,778, mais dans laquelle un atome M' est substitué à un atome B. Cette dernière formule a pour intérêt de conduire à des rapports atomiques entre l'élément du groupe VIII et du groupe VIB pouvant aller jusqu'à 0,5 et donc des phases actives mieux promues.

Le brevet FR 2,315,721 a mis en évidence l'intérêt de l'utilisation d'hétéropolycomposés de formule $Ni_{x+y/2}AW_{11-y}O_{39-5/2y}$, $zH_2O$ et plus particulièrement l'emploi des hétéropolycomposés de formule $Ni_4SiW_{11}O_{39}$ et de

formule $Ni_5SiW_9O_{34}$ conduisant à des performances catalytiques inattendues en hydrocraquage et en hydrotraitement.

Dans tous les cas, les équipes ont cherché par l'utilisation de sels de nickel d'hétéropolymolybdates ou d'hétéropolytungstates à favoriser l'interaction métal-promoteur en les plaçant dans la même entité moléculaire, ce qui permettrait de contrôler le taux de promotion du catalyseur sulfuré et ainsi d'en augmenter le nombre de sites actifs.

Enfin, l'utilisation de ces polyoxométallates piégés dans des silices mésostructurées a également été révélée dans les brevets FR 2,969,647 et FR 2,969,645. Les catalyseurs des inventions ont montré des performances en hydrotraitement de gazole et en hydrocraquage très intéressantes comparativement à des catalyseurs préparés de manière conventionnelle (imprégnation de polyoxométallates sur des supports mésoporeux).

Préparation des catalyseurs d'hydrotraitement ou d'hydrocraquage avec ajout de molécules organiques

L'ajout d'un composé organique sur les catalyseurs d'hydrotraitement pour améliorer leur activité est maintenant bien connu de l'Homme du métier. De nombreux brevets protègent l'utilisation de différentes gammes de composés organiques, tels que les mono,-di-ou polyalcools éventuellement éthérifiés (WO96/41848, WO01/76741, US 4,012,340, US 3,954,673, EP601722). Des catalyseurs modifiés avec des monoesters en C2-C14 sont décrits dans les demandes de brevet EP 0,466,568 et EP 1 046 424, cependant ces modifications ne permettent pas toujours d'accroître suffisamment les performances du catalyseur pour faire face aux spécifications concernant les teneurs en soufre des carburants qui ne cessent de devenir de plus en plus contraignantes pour les raffineurs.

Pour remédier à cela, le brevet FR 2,880,823 décrit l'utilisation d'un catalyseur comprenant des métaux des groupes VIB et VIII, un oxyde réfractaire comme support, et un composé organique, comportant au moins 2 fonctions ester carboxylique de formule R1-O-CO-R2-CO-O-R1 ou R1-CO-O-R2-O-CO-R1 ou encore le succinate de dialkyle C1-C4 avec de l'acide acétique dans le brevet FR 2,953,740.

D'autres brevets dans l'art antérieur décrivent un gain d'activité lié à l'utilisation combinée d'un acide organique ou d'un alcool sur un catalyseur d'hydrotraitement, comme dans la demande de brevet publiée sous le n° JP1995-136523 de KK Japan Energy ou lié à l'utilisation d'oligosaccharide cyclique comme dans le brevet 2,963,360 par exemple.

Même si les gains d'activité sont parfois mal expliqués, l'intérêt de l'utilisation de molécules organiques lors de la préparation des catalyseurs d'hydrotraitement et d'hydrocraquage n'est plus à démontrer, cependant ces préparations restent limitées par le nombre d'étapes et par des molécules organiques à imprégner insolubles dans les solutions aqueuses classiquement utilisées.

Préparation des catalyseurs d'hydrotraitement et d'hydrocraquage avec des précurseurs mononucléaires (précurseur ne contenant qu'un atome de métal dans sa structure)

Les préparations concernant des catalyseurs supportés à partir de précurseurs différents des polyoxométallates, ne présentant qu'un seul atome de molybdène ou de tungstène dans leur structure, sont connues depuis longtemps, mais restent peu nombreuses.

Dès les années 1980, il a été montré que l'utilisation de précurseurs organométalliques à base de Mo ou de W de type allyl ($WR_4$ où R = $C_4H_7$) déposés sur $SiO_2$ permettait de générer des catalyseurs NiW ou NiMo dont les activités intrinsèques (activité ramenée par atome de Mo ou de W) en hydrodésulfuration étaient jusqu'à 4 fois supérieures à celles des catalyseurs préparés de manière plus conventionnelle (utilisation d'heptamolybdate d'ammonium $(NH_4)_6Mo_7O_{24}$, $6H_2O$ dans le cas des catalyseurs préparés à partir de Mo ou l'acide tungstique, $H_2WO_4$ dans le cas des catalyseurs préparés à partir de W) (Yermakov et al., Journal of Molecular Catalysis, 1981, 205-214, Yermakov, Journal of molecular catalysis, 21, 1983, 35-55 et Yermakov et al., Applied Catalysis 11, 1984, 1-13) . Les catalyseurs préparés sur silice à partir de précurseurs organométalliques restent cependant plus actifs (activité ramenée par atome de Mo ou de W) que les catalyseurs préparés sur alumine.

Dans les années 1990, ont été préparés des catalyseurs de type CoMo sur alumine à partir de sels de thiomolybdate (bis(tetrabutylammonium) tetrathimolybdate $(TBA)_2MoS_4$) et l'intérêt de leur utilisation a été démontré pour des applications en hydrodésulfuration (Halbert et al. Journal of Catalysis 130, 1991, 116-129).

En 2008, l'intérêt d'utiliser le molybdenedioxodiacétylactéonate sur une alumine mésoporeuse organisée pour la préparation de catalyseurs d'hydrotraitement a été démontré (Kaluza et al., Applied Catalysis A : General, 351, 2008, 93-101). Ces travaux ont alors révélé que les catalyseurs CoMo et NiMo préparés à partir de ce précurseur étaient plus hydrodésulfurants que des catalyseurs commerciaux. Le brevet EP 0,178,711 décrit la préparation de catalyseurs d'hydrotraitement sur silice à partir de solutions contenant des halogénures de Mo, de manière préférée le MoCl5, en présence d'halogénure de nickel et/ou de cobalt, de manière préférée le chlorure de nickel et/ou le chlorure de cobalt hexahydrate, $NiCl_2(H_2O)_6$ et $CoCl_2(H_2O)_6$ respectivement, dans un solvant de type nitrile, avec éventuellement, en plus, un solvant chloré.

Le brevet US 5,137,859, quant à lui, décrit la préparation de catalyseurs utilisés pour l'hydrodésulfuration de coupes pétrolières hydrocarbonées sur un support aluminique à partir d'un composé choisi parmi les alcoxydes ou les composés chélatants ou les glycoxydes de molybdène ou de chrome et d'un composé choisi parmi les alcoxydes ou les composés chélatants ou les glycoxydes de nickel ou de cobalt solubilisés dans un solvant organique choisi parmi les alcools, les éthers, les cétones et les composés aromatiques. Le catalyseur oxyde fraîchement imprégné subit obligatoirement une étape de séchage à une température d'environ 150°C en présence ou en l'absence d'oxygène et obligatoirement une étape de calcination à une température au moins égale à 200°C sous une atmosphère contenant de l'oxygène. Ces traitements peuvent favoriser la dénaturation des précurseurs greffés par calcination de la partie carbonée et éventuellement générer la polycondensation des espèces alcoxydes soit à cause de l'eau présente dans le gaz de traitement thermique, soit à cause de l'eau qui serait libérée lors de la calcination des groupements carbonés. Dès lors, on peut supposer perdre la dispersion initialement apportée lors du greffage des espèces intactes et générer moins de sites actifs sur la surface après sulfuration.

Les travaux de recherche de la demanderesse l'ont donc conduite à préparer des catalyseurs d'hydrogénation à partir de molybdène et éventuellement d'au moins un élément du groupe VIII, notamment le nickel en modifiant la composition chimique et structurale des espèces métalliques précurseurs des phases actives, afin de modifier les interactions entre le support et ces précurseurs afin de mieux sulfurer le tungstène reconnu comme étant difficile à sulfurer, mais aussi afin de modifier les interactions entre le support et la phase active sulfure du catalyseur pour mieux la disperser. En particulier, les travaux de la demanderesse l'ont conduite à utiliser des précurseurs mononucléaires à base de molybdène, sous leur forme monomérique ou dimérique, présentant au moins une liaison Mo=O ou Mo-OR ou au moins une liaison Mo=S ou Mo-SR où [R = $C_xH_y$ où x $\geq$ 1 et (x-1) $\leq$ y $\leq$ (2x+1) ou R = Si(OR')$_3$ ou R = Si(R')$_3$ où R' = $C_{x'}$-$H_{y'}$ où x' $\geq$ 1 et (x'-1) $\leq$ y' $\leq$ (2x'+1)], comme précurseurs particuliers de la phase active des catalyseurs utilisés dans les réactions d'hydrogénation des procédés d'hydrotraitement et des procédés d'hydrocraquage de charges hydrocarbonées selon l'invention.

La demanderesse a donc mis en évidence qu'un catalyseur supporté préparé à partir d'au moins un précurseur mononucléaire à base de Mo, sous sa forme monomérique ou dimérique, et présentant au moins une liaison Mo=O ou Mo-OR ou au moins une liaison Mo=S ou Mo-SR où [R = $C_xH_y$ où x $\geq$ 1 et (x-1) $\leq$ y $\leq$ (2x+1) ou R = Si(OR')$_3$ ou R = Si(R')$_3$ où R' = $C_{x'}$Hy$_'$ où x' $\geq$ 1 et (x'-1) $\leq$ y' $\leq$ (2x'+1)], selon un mode de préparation particulier, présentait une sulfuration améliorée et une activité catalytique améliorée par rapport à des catalyseurs préparés à partir de précurseurs standards tels que les polyoxométallates, ledit catalyseur ayant été avantageusement préalablement sulfuré puis mis en oeuvre dans un procédé d'hydrotraitement ou d'hydrocraquage.

## Description de l'invention

### Objets de l'invention

L'invention concerne un procédé de préparation d'un catalyseur supporté à partir d'au moins un précurseur mononucléaire à base de molybdène, sous sa forme monomérique ou dimérique, présentant au moins une liaison Mo=O ou Mo-OR ou au moins une liaison Mo=S ou Mo-SR où [R = $C_xH_y$ où x $\geq$ 1 et (x-1) $\leq$ y $\leq$ (2x+1) ou R = Si(OR')$_3$ ou R = Si(R')$_3$ où R' = $C_{x'}H_{y'}$ où x' $\geq$ 1 et (x'-1) $\leq$ y' $\leq$ (2x'+1)], et éventuellement d'au moins un élément du groupe VIII.

L'invention concerne également le catalyseur susceptible d'être préparé par ledit procédé de préparation.

L'invention concerne enfin l'utilisation dudit catalyseur susceptible d'être ainsi préparé dans des réactions d'hydrogénation, notamment dans les procédés d'hydrotraitement et d'hydrocraquage.

### Résumé de l'invention

L'invention concerne un procédé de préparation d'un catalyseur renfermant au moins un support, éventuellement au moins un métal du groupe VIII de la classification périodique des éléments, et au moins du molybdène dans lequel :

- on introduit le molybdène dans un solvant organique A sur le support, sous la forme d'au moins un composé précurseur mononucléaire à base de Mo, sous sa forme monomérique ou dimérique, présentant au moins une liaison Mo=O ou Mo-OR ou au moins une liaison Mo=S ou Mo-SR où [R = $C_xH_y$ où x $\geq$ 1 et (x-1) $\leq$ y $\leq$ (2x+1) ou R = Si(OR')$_3$ ou R = Si(R')$_3$ où R' = $C_{x'}H_{y'}$ où x' $\geq$ 1 et (x'-1) $\leq$ y' $\leq$ (2x'+1)],
- et on effectue un traitement thermique du support ainsi imprégné par séchage à une température inférieure à 200°C.

Ledit séchage peut être effectué sous atmosphère anhydre ou sous vide primaire ou sous vide secondaire ou sous flux de gaz inerte.

De préférence, ledit séchage est effectué sous vide et à température ambiante.

Le métal du groupe VIII est avantageusement choisi parmi le cobalt, le fer ou le nickel.

Le métal du groupe VIII est de préférence le nickel.

Le précurseur du molybdène est avantageusement un précurseur mononucléaire à base de Mo, utilisé sous sa forme monomérique ou dimérique, de formule

$$Mo(=O)_n(=S)_{n'}(OR)_a(SR')_b(L1)_c(L2)_d(L3)_e(L4)_f(L5)_g$$

où R = $C_xH_y$ où x ≥ 1 et (x-1) ≤ y ≤ (2x+1) ou R = Si(OR")$_3$ ou R = Si(R")$_3$ où R" = $C_{x'}H_{y'}$, où [x" ≥ 1 et (x"-1) ≤ y" ≤ (2x"+1)], où R' = $C_{x'}H_{y'}$ où x' ≥ 1 et (x'-1) ≤ y' ≤ (2x'+1) ou R' = Si(OR''')$_3$ ou R' = Si(R''')$_3$ où R''' = $C_{x'''}H_{y'''}$ où [x''' ≥ 1 et (x'''-1) ≤ y''' ≤ (2x'''+1)],

où 0 ≤ n+n' ≤ 2 et 0 ≤ n ≤ 2 et 0 ≤ n' ≤ 2,

où, si n = n' = 0, alors (a#0 ou b#0) et [(a+b+c+d+e+f+g = 6 et 0 ≤ a ≤ 6, 0 ≤ b ≤ 6, 0 ≤ c ≤ 6, 0 ≤ d ≤ 6, 0 ≤ e ≤ 6, 0 ≤ f ≤ 6, 0 ≤ g ≤ 6, ou (a+b+c+d+e+f+g = 5 et 0 ≤ a ≤ 5, 0 ≤ b ≤ 5, 0 ≤ c ≤ 5, 0 ≤ d ≤ 5, 0 ≤ e ≤ 5, 0 ≤ f ≤ 5, 0 ≤ g ≤ 5), ou (a+b+c+d+e+f+g = 4 et 0 ≤ a ≤ 4, 0 ≤ b ≤ 4, 0≤ c ≤ 4, 0 ≤ d ≤ 4, 0 ≤ e ≤ 4, 0 ≤ f ≤ 4, 0 ≤ g ≤ 4)]

où, si [(n=1 et n' = 0) ou (n' = 1 et n = 0)], alors [a+b+c+d+e+f+g = 4 et 0 ≤ a ≤ 4, 0 ≤ b ≤ 4, 0 ≤ c ≤ 4, 0 ≤ d ≤ 4, 0 ≤ e ≤ 4, 0 ≤ f ≤ 4, 0 ≤ g ≤ 4)] ou [(a+b+c+d+e+f+g = 3 et 0 ≤ a ≤ 3, 0 ≤ b ≤ 3, 0 ≤ c ≤ 3, 0 ≤ d ≤ 3, 0 ≤ e ≤ 3, 0 ≤ f ≤ 3, 0 ≤ g ≤ 3)]

où, si [n+n'=2 et 0 ≤ n ≤ 2 et 0 ≤ n' ≤ 2], alors (a+b+c+d+e+f+g = 2 et 0 ≤ a ≤ 2, 0 ≤ b ≤ 2, 0 ≤ c ≤ 2, 0 ≤ d ≤ 2, 0 ≤ e ≤ 2, 0 ≤ f ≤ 2, 0 ≤ g ≤ 2),

avec (L1), (L2), (L3), (L4) et (L5) choisis parmi les ligands de type THF, dimethyl ether, dimethylsulfure, P(CH$_3$)$_3$, allyl, aryl, halogénés, amine, acétate, acétylacétonate, halogénure, hydroxyde, -SH,...ou tout autre ligand bien connu de l'homme du métier.

De préférence, le précurseur de molybdène est choisi parmi Mo(OEt)$_5$, Mo(OEt)$_6$, Mo(=O)(OEt)$_4$, Mo(=S)(OEt)$_4$, Mo(=S)(SEt)$_4$, Mo(=O)$_2$(OEt)$_2$, Mo(OC$_6$H$_5$)$_6$, Mo(SEt)$_5$, Mo(SEt)$_6$, Mo(OEt)(SEt)$_4$, Mo(OEt)$_2$(SEt)$_3$, Mo(OEt)$_3$(SEt)$_2$, Mo(OEt)$_4$(SEt), Mo(=O)(OEt)$_3$(acac) avec Et = CH$_2$CH$_3$ (groupement éthyle) et acac = (CH$_3$COCHCOCH$_3$)-(acétylacétonate) sous leur forme monomérique ou dimérique.

Le molybdène, et le ou les métaux du groupe VIII éventuels peuvent être introduits simultanément ou successivement.

Avantageusement le procédé de préparation comprend au moins une étape finale de sulfuration en phase gaz, in situ et/ou ex situ.

Plus particulièrement, le procédé de préparation peut comprendre au moins les étapes suivantes :

a) imprégnation par mise en contact d'une solution S comprenant le solvant organique A et au moins ledit précurseur mononucléaire à base de molybdène, sous sa forme monomérique ou dimérique, présentant au moins une liaison Mo=O ou Mo-OR ou au moins une liaison Mo=S ou Mo-SR où [R = $C_xH_y$ où x ≥ 1 et (x-1) ≤ y ≤ (2x+1) ou R = Si(OR)$_3$ ou R = Si(R')$_3$ où R' = $C_{x'}H_{y'}$ où x' ≥ 1 et (x'-1) ≤ y' ≤ (2x'+1)], avec un support minéral poreux, préalablement calciné sous vide primaire ou sous vide secondaire ou sous flux de gaz inerte ;

b) maturation sous atmosphère anhydre ;

c) séchage du support imprégné à une température inférieure à 200°C sous atmosphère anhydre ou sous vide primaire ou sous vide secondaire ou sous flux de gaz inerte.

d) sulfuration ex-situ sous un mélange H$_2$S/H$_2$ ou H$_2$S/N$_2$ contenant au moins 5% volumique d'H$_2$S dans le mélange à une température égale ou supérieure à la température ambiante.

Le métal du groupe VIII éventuel peut être introduit à l'étape a) dans la même solution S que le précurseur de molybdène, ou après le séchage c) dans une étape de post-imprégnation a2) à l'aide d'une solution utilisant un solvant organique B, ou après l'étape d) de sulfuration, dans une étape de post-imprégnation a3) à l'aide d'une solution aqueuse ou organique.

L'invention concerne également un catalyseur susceptible d'être préparé selon ledit procédé de préparation.

De préférence, ledit catalyseur comprend une teneur en molybdène comprise entre 4 et 30 % poids et une teneur en métal(aux) du groupe VIII comprise entre 0,1 à 8% poids par rapport au poids total de catalyseur.

L'invention concerne également l'utilisation dudit catalyseur dans les réactions d'hydrogénation de charges hydrocarbonées, de préférence pour l'hydrotraitement ou l'hydrocraquage.

## Description détaillée de l'invention

L'invention concerne un procédé de préparation d'un catalyseur permettant des réactions d'hydrogénation dans les procédés d'hydrotraitement et d'hydrocraquage, préparé à partir d'au moins un précurseur mononucléaire à base de molybdène, sous sa forme monomérique ou dimérique, présentant au moins une liaison Mo=O ou Mo-OR ou au moins une liaison Mo=S ou Mo-SR où [R = $C_xH_y$ où x ≥ 1 et (x-1) ≤ y ≤ (2x+1) ou R = Si(OR')$_3$ ou R = Si(R')$_3$ où R' = $C_{x'}H_{y'}$ où x' ≥ 1 et (x'-1) ≤ y' ≤ (2x'+1)], et éventuellement d'au moins un élément du groupe VIII. Lesdits précurseurs sont déposés par tout moyen connu de l'homme du métier sur un support oxyde adapté au procédé dans lequel il est utilisé, ledit catalyseur étant séché à une température inférieure à 200°C et avantageusement sulfuré avant d'être mis en oeuvre dans ledit procédé.

Un des avantages de la présente invention réside donc dans une préparation de catalyseurs d'hydrotraitement à base de molybdène innovante permettant une meilleure dispersion de par le greffage et la préservation des précurseurs à la surface du support, même sur un support silicique. Ces améliorations permettent de générer potentiellement plus de phase active de type sulfure et donc potentiellement plus de sites actifs pouvant réaliser les réactions d'hydrogénation ou d'hydrocraquage souhaitées et donc des activités plus importantes pour les catalyseurs selon l'invention comparativement à ceux rencontrés dans la littérature ou des activités identiques aux catalyseurs conventionnels, mais avec deux fois moins d'atomes de métal sur le catalyseur.

De manière préférée, ladite préparation comprend au moins les étapes suivantes :

a) une étape d'imprégnation par mise en contact d'une solution comprenant un solvant organique A et au moins un précurseur mononucléaire à base de Mo, sous sa forme monomérique ou dimérique, présentant au moins une liaison Mo=O ou Mo-OR ou au moins une liaison Mo=S ou Mo-SR où [R = $C_xH_y$ où $x \geq 1$ et $(x-1) \leq y \leq (2x+1)$ ou R = $Si(OR')_3$ ou R = $Si(R')_3$ où R' = $C_{x'}H_{y'}$ où $x' \geq 1$ et $(x'-1) \leq y' \leq (2x'+1)$], avec un support minéral poreux, avantageusement préalablement calciné sous vide primaire ou sous vide secondaire ou sous flux de gaz inerte pour évacuer l'eau éventuellement physisorbée sur ledit support,

b) une étape de maturation ;

c) une étape de séchage du support imprégné, à une température inférieure à 200°C, sous atmosphère anhydre ou sous vide primaire ou sous vide secondaire ou sous flux de gaz inerte ;

d) une étape de sulfuration, de préférence réalisée ex-situ sous un mélange $H_2S/H_2$ ou $H_2S/N_2$ contenant au moins 5% volumique d'$H_2S$ dans le mélange à une température égale ou supérieure à la température ambiante.

Le ou les éléments du groupe VIII éventuels, par la suite indiqués comme étant le(s) promoteur(s), peu(ven)t être introduit(s) en solution soit :

i) à l'étape d'imprégnation a) en co-imprégnation avec le précurseur mononucléaire à base de molybdène,

ii) après l'étape de séchage c), dans une étape dite de post-imprégnation a2) à l'aide d'une solution utilisant un solvant organique B. Dans ce cas, une seconde étape de maturation b2) et un second séchage c2) à une température inférieure à 200 °C sont nécessaires et peuvent être réalisés dans les mêmes conditions que les conditions décrites lors des étapes b) et c)

iii) après l'étape d), dans une étape de post-imprégnation a3) à l'aide d'une solution aqueuse ou d'une solution organique. Dans ce cas, il est nécessaire d'ajouter une nouvelle étape de maturation b3), une nouvelle étape de séchage à une température inférieure à 200°C c3) et une nouvelle étape de sulfuration d2) avant d'utiliser le catalyseur dans le procédé d'hydrotraitement ou d'hydrocraquage selon l'invention.

Le précurseur mononucléaire à base de Mo, utilisé sous sa forme monomérique ou dimérique, selon l'invention a avantageusement pour formule

$$Mo(=O)_n(=S)_{n'}(OR)_a(SR')_b(L1)_c(L2)_d(L3)_e(L4)_f(L5)_g$$

où R = $C_xH_y$ où $x \geq 1$ et $(x-1) \leq y \leq (2x+1)$ ou R = $Si(OR'')_3$ ou R = $Si(R'')_3$ où R'' = $C_{x'}H_{y'}$ où $[x'' \geq 1$ et $(x''-1) \leq y'' \leq (2x''+1)]$,

où R' = $C_{x'}H_{y'}$ où $x' \geq 1$ et $(x'-1) \leq y' \leq (2x'+1)$ ou R' = $Si(OR''')_3$ ou R' = $Si(R''')_3$ où R''' = $C_{x'''}H_{y'''}$ où $[x''' \geq 1$ et $(x'''-1) \leq y''' \leq (2x'''+1)]$,

où $0 \leq n+n' \leq 2$ et $0 \leq n \leq 2$ et $0 \leq n' \leq 2$,

où, si n = n' = 0, alors (a#0 ou b#0) et [(a+b+c+d+e+f+g = 6 et $0 \leq a \leq 6$, $0 \leq b \leq 6$, $0 \leq c \leq 6$, $0 \leq d \leq 6$, $0 \leq e \leq 6$, $0 \leq f \leq 6$, $0 \leq g \leq 6$, ou (a+b+c+d+e+f+g = 5 et $0 \leq a \leq 5$, $0 \leq b \leq 5$, $0 \leq c \leq 5$, $0 \leq d \leq 5$, $0 \leq e \leq 5$, $0 \leq f \leq 5$, $0 \leq g \leq 5$), ou (a+b+c+d+e+f+g = 4 et $0 \leq a \leq 4$, $0 \leq b \leq 4$, $0 \leq c \leq 4$, $0 \leq d \leq 4$, $0 \leq e \leq 4$, $0 \leq f \leq 4$, $0 \leq g \leq 4$)]

où, si [(n=1 et n' = 0) ou (n' = 1 et n = 0)], alors [a+b+c+d+e+f+g = 4 et $0 \leq a \leq 4$, $0 \leq b \leq 4$, $0 \leq c \leq 4$, $0 \leq d \leq 4$, $0 \leq e \leq 4$, $0 \leq f \leq 4$, $0 \leq g \leq 4$)] ou [(a+b+c+d+e+f+g = 3 et $0 \leq a \leq 3$, $0 \leq b \leq 3$, $0 \leq c \leq 3$, $0 \leq d \leq 3$, $0 \leq e \leq 3$, $0 \leq f \leq 3$, $0 \leq g \leq 3$)]

où, si [n+n'=2 et $0 \leq n \leq 2$ et $0 \leq n' \leq 2$], alors (a+b+c+d+e+f+g = 2 et $0 \leq a \leq 2$, $0 \leq b \leq 2$, $0 \leq C \leq 2$, $0 \leq d \leq 2$, $0 \leq e \leq 2$, $0 \leq f \leq 2$, $0 \leq g \leq 2$).

avec (L1), (L2), (L3), (L4) et (L5), des ligands bien connus de l'Homme du métier et de type THF, dimethyl ether, dimethylsulfure, $P(CH_3)_3$, allyl, aryl, halogénés (choisis parmi les fluorés, les chlorés, les bromés, amine, acétate, acétylacétonate, halogénure, hydroxyde, - SH,... De manière préférée, les ligands sont choisis parmi l'acétylacétonate, le THFet le dimethyl ether.

De manière préférée, les précurseurs selon l'invention ne contiennent pas de ligand (L1), (L2), (L3), (L4) et (L5).

De manière très préférée, les précurseurs selon l'invention sont choisis parmi les composés suivants : $Mo(OEt)_5$, $Mo(OEt)_6$, $Mo(=O)(OEt)_4$, $Mo(=S)(OEt)_4$, $Mo(=S)(SEt)_4$, $Mo(=O)_2(OEt)_2$, $Mo(OC_6H_5)_6$, $Mo(SEt)_5$, $Mo(SEt)_6$, $Mo(OEt)(SEt)_4$, $Mo(OEt)_2(SEt)_3$, $Mo(OEt)_3(SEt)_2$, $Mo(OEt)_4(SEt)$, $Mo(=O)(OEt)_3(acac)$ avec Et = $CH_2CH_3$ (groupement éthyle) et acac = $(CH_3COCHCOCH_3)^-$ (acétylacétonate) sous leur forme monomérique ou dimérique.

De manière encore plus préférée, le précurseur de molybdène est le $Mo(OEt)_5$.

La teneur en molybdène Mo est généralement comprise entre 4 et 30% poids par rapport au catalyseur final, et de manière préférée entre 7 et 25% poids par rapport au catalyseur final, obtenu après la dernière étape de préparation, avant la mise en oeuvre dans le procédé d'hydrotraitement ou le procédé d'hydrocraquage.

La densité surfacique qui correspond à la quantité d'atomes de molybdène Mo déposés par unité surfacique de support sera avantageusement comprise entre 0,5 et 8 atomes de Mo par nanomètres carré de support et de manière préférée entre 1 et 7 atomes de Mo par nanomètres carré de support.

L'étape a) dite de mise en contact de la solution et du support est une imprégnation. Les imprégnations sont bien connues de l'Homme de l'art. La méthode d'imprégnation selon l'invention est choisie parmi l'imprégnation à sec, l'imprégnation en excès, les imprégnations successives. La méthode dite d'imprégnation à sec est avantageusement utilisée.

Le solvant organique A utilisé à l'étape a) est généralement un alcane, un alcool, un éther, une cétone, un solvant chloré ou un composé aromatique. Le cyclohexane et le n-hexane sont utilisés de manière préférée.

L'étape b) est une étape de maturation destinée à laisser diffuser les espèces à coeur du support. Elle est réalisée avantageusement sous atmosphère anhydre (sans eau), de préférence entre 30 minutes et 24h à température ambiante. L'atmosphère doit être de préférence anhydre afin de ne pas polycondenser les précurseurs préalablement imprégnés.

Le séchage réalisé au cours de l'étape c) est destiné à évacuer le solvant A d'imprégnation. L'atmosphère doit être de préférence anhydre (sans eau) afin de ne pas polycondenser lesdits précurseurs préalablement imprégnés. La température ne doit pas excéder 200°C afin de garder intacts lesdits précurseurs greffés ou déposés à la surface du support. De préférence, la température n'excédera pas 120°C. De manière très préférée, le séchage s'effectue sous vide, à température ambiante. Cette étape peut s'effectuer alternativement par le passage d'un flux gazeux inerte.

L'étape d) de sulfuration peut être réalisée avantageusement ex-situ à l'aide d'un mélange gazeux $H_2S/H_2$ ou $H_2S/N_2$ contenant au moins 5% volumique d'$H_2S$ dans le mélange à une température égale ou supérieure à la température ambiante, sous une pression totale égale ou supérieure à 1 bar pendant au moins 2h. De manière préférée, la température de sulfuration est de 250°C. De manière très préférée, la température de sulfuration est de 350 °C.

L'étape d) de sulfuration peut également, ou en plus de l'étape d) réalisée ex-situ, être réalisée in situ, au début de la mise en oeuvre du procédé catalytique utilisant le catalyseur, par exemple un procédé d'hydrotraitement ou d'hydrocraquage, par tout procédé de sulfuration bien connu de l'homme du métier, comme décrit ci-après.

Les éléments du groupe VIII préférés sont des éléments non nobles : ils sont choisis parmi le Ni, le Co et le Fe. De manière préférée, les éléments du groupe VIII sont le cobalt et le nickel. De manière très privilégiée, l'élément du groupe VIII est le nickel. Le métal du groupe VIII est introduit sous la forme de sels, de composés chélatants, d'alcoxydes ou de glycoxydes, et de manière préférée, sous la forme d'acétylacétonate ou d'acétate.

Si le promoteur est introduit comme il est décrit dans l'invention en i) et en ii), les composés contenant l'élément du groupe VIII sont de manière privilégiée les composés soufrés, les composés oxygénés, les composés chélatants, les alcoxydes et les glycoxydes. De manière préférée, il est introduit sous la forme d'acétylacétonate ou d'acétate.

Si le promoteur est introduit comme il est décrit dans l'invention en iii), les composés contenant l'élément du groupe VIII peuvent être introduits sous la forme de sels, de composés soufrés, de composés oxygénés, de composés chélatants, d'alcoxydes et de glycoxydes. De manière préférée, il est introduit sous la forme sous la forme d'acétylacétonate ou d'acétate.

Les sources d'éléments du groupe VIII qui peuvent avantageusement être utilisées sous forme de sels, sont bien connues de l'Homme du métier. Ils sont choisis parmi les nitrates, les sulfates, les hydroxydes, les phosphates, les halogénures choisis parmi les chlorures, les bromures et les fluorures.

Les éléments promoteurs du groupe VIII sont avantageusement présents dans le catalyseur à des teneurs comprises entre 0,1 et 8% poids, de préférence entre 0,5 et 5% poids par rapport au catalyseur final obtenu après la dernière étape de préparation, avant la mise en oeuvre dans le procédé d'hydrotraitement ou le procédé d'hydrocraquage.

Le solvant organique B utilisé lorsque le promoteur est introduit après l'étape c), dans une étape dite de post-imprégnation est généralement un alcane, un alcool, un éther, une cétone, un composé chloré ou un composé aromatique. Le toluène, le benzène, le dichlorométhane, le tétrahydrofurane, le cyclohexane, le n-hexane, l'éthanol, le méthanol et l'acétone sont utilisés de manière préférée.

Le solvant utilisé pour l'imprégnation du promoteur (élément du groupe VIII) dans le cas de l'étape iii) correspond soit au solvant organique B dans le cas de l'utilisation de précurseurs non salins et de l'eau ou un alcool lorsque les précurseurs sont salins.

La fonction hydro-deshydrogénante dudit précurseur catalytique est assurée par le molybdène et éventuellement par au moins un élément du groupe VIII. Avantageusement, la fonction hydro-déhydrogénante est choisie dans le groupe formé par les combinaisons des éléments nickel-molybdène ou cobalt-molybdène ou nickel-cobalt-molybdène.

Le support du catalyseur de l'invention est un support minéral poreux qui comprend avantageusement au moins de l'aluminium et/ou au moins du silicium.

De préférence, ledit support comprend au moins un oxyde d'aluminium ou au moins un oxyde de silicium. Ledit support peut avantageusement être acide ou non. Ledit support peut avantageusement être mésostructuré ou non.

Ledit support minéral poreux peut avantageusement être choisi parmi les alumines de transition, les alumines dopées, de préférence au phosphore, au bore et/ou au fluor, la silicalite et les silices, les aluminosilicates, de préférence amorphes ou mal cristallisés, les tamis moléculaires cristallisés non zéolithiques tels que les silicoaluminophosphates, les aluminophosphates, les ferrosilicates, les silicoaluminates de titane, les borosilicates, les chromosilicates et les aluminophosphates de métaux de transition, seuls ou en mélange. Dans le cas où ledit support minéral poreux est choisi parmi les alumines de transition, la silicalite et les silices telles que par exemple les silices mésoporeuses, ledit support n'est pas acide. Par alumine de transition, on entend par exemple une alumine phase alpha, une alumine phase delta, une alumine phase gamma ou un mélange d'alumine de ces différentes phases.

Dans le cas où ledit support minéral poreux est choisi parmi les aluminosilicates, de préférence amorphes ou mal cristallisés, les tamis moléculaires cristallisés non zéolithiques tels que les silicoaluminophosphates, les aluminophosphates, les ferrosilicates, les silicoaluminates de titane, les borosilicates, les chromosilicates et les aluminophosphates de métaux de transition, les alumines dopées, de préférence au phosphore, au bore et/ou au fluor, ledit support est acide. Toute silice-alumine connue ou tout aluminosilicate connu de l'homme de l'art convient pour l'invention.

Lorsque ledit support minéral poreux est dit mésostructuré, il comprend alors des particules élémentaires organisées à l'échelle des mésopores du matériau selon l'invention, c'est-à-dire une porosité organisée à l'échelle des pores ayant un diamètre uniforme compris entre 1,5 et 50 nm, de préférence entre 1,5 et 30 nm et de manière encore plus préférée entre 4 et 20 nm et répartis de façon homogène et régulière dans chacune desdites particules (mésostructuration). La matière située entre les mésopores de la particule élémentaire mésostructurée est amorphe et forme des parois, ou murs, dont l'épaisseur est comprise entre 1 et 30 nm et de préférence entre 1 et 10 nm. L'épaisseur des parois correspond à la distance séparant un premier mésopore d'un second mésopore, le second mésopore étant le pore le plus proche dudit premier mésopore. L'organisation de la mésoporosité décrite ci-dessus conduit à une structuration de ladite particule constitutive dudit support, laquelle peut être hexagonale, vermiculaire ou cubique et de façon préférée hexagonale. De préférence, ledit support minéral poreux mésostructuré est choisi parmi la silice et la silice alumine.

Le support minéral poreux selon l'invention, qu'il soit acide ou non, mésostructuré ou non, peut également avantageusement renfermer, en plus d'au moins un des composés oxydes cités ci-dessus, au moins une zéolithe et en particulier mais de façon non restrictive celles répertoriées dans "Atlas of zéolite framework types", 6th revised Edition, 2007, Ch. Baerlocher, L. B. L.McCusker, D. H. Olson". Les cristaux zéolithiques peuvent être choisis parmi les zéolithes IZM-2, ZSM-5, ZSM-12, ZSM-48, ZSM-22, ZSM-23, ZBM-30, EU-2, EU-11, Silicalite, Bêta, zéolithe A, Faujasite, Y, USY, VUSY, SDUSY, Mordénite, NU-10, NU-87, NU-88, NU-86, NU-85, IM-5, IM-12, IM-16, Ferriérite et EU-1. De manière très préférée, les cristaux zéolithiques peuvent être choisis parmi les zéolithes de type structural MFI, BEA, FAU, et LTA. Des cristaux zéolithiques différents et notamment des zéolithes de type structural différent peuvent être présentes dans le support minéral poreux constituant le matériau selon l'invention. En particulier, le support minéral poreux selon l'invention peut comprendre de manière avantageuse au moins des premiers cristaux zéolithiques dont la zéolithe est choisie parmi les zéolithes IZM-2, ZSM-5, ZSM-12, ZSM-48, ZSM-22, ZSM-23, ZBM-30, EU-2, EU-11, Silicalite, Bêta, zéolithe A, Faujasite, Y, USY, VUSY, SDUSY, Mordénite, NU-10, NU-87, NU-88, NU-86, NU-85, IM-5, IM-12, IM-16, Ferriérite et EU-1, de préférence parmi les zéolithes de type structural MFI, BEA, FAU, et LTA et au moins des seconds cristaux zéolithiques dont la zéolithe est différente de celle des premiers cristaux zéolithiques et est choisie parmi les zéolithes IZM-2, ZSM-5,ZSM-12, ZSM-48, ZSM-22, ZSM-23, ZBM-30, EU-2, EU-11, Silicalite, Bêta, zéolithe A, Faujasite, Y, USY, VUSY, SDUSY, Mordénite, NU-10, NU-87, NU-88, NU-86, NU-85, IM-5, IM-12, IM-16, Ferriérite et EU-1, de préférence parmi les zéolithes de type structural MFI, BEA, FAU, et LTA. Les cristaux zéolithiques comprennent avantageusement au moins une zéolithe soit entièrement silicique soit contenant, outre du silicium, au moins un élément T choisi parmi l'aluminium, le fer, le bore, l'indium, le gallium et le germanium, de préférence l'aluminium.

Le support minéral poreux peut également avantageusement renfermer, en plus d'au moins un des composés oxydes cités ci-dessus, au moins une argile simple synthétique ou naturelle de type phyllosilicate 2:1 dioctaédrique ou phyllosilicate 3:1 trioctaédrique telles que la kaolinite, l'antigorite, la chrysotile, la montmorillonnite, la beidellite, la vermiculite, le talc, l'hectorite, la saponite, la laponite. Ces argiles peuvent être éventuellement délaminées.

De préférence, ledit support minéral poreux est choisie parmi l'alumine et la silice alumine mésoporeuses, prises seules ou en mélange ou les silices et les silices alumines mésostructurées prises seules ou en mélange.

Le catalyseur peut être utilisé sous toutes les formes connues par l'Homme de l'art : il peut se présenter sous forme de poudre, sous forme de bille ou sous forme d'extrudés cylindriques, trilobes ou quadrilobes. Différentes mise en forme peuvent être mélangées.

Conformément à l'invention, ledit catalyseur est avantageusement partiellement sulfuré grâce à au moins une étape de sulfuration sous phase gaz décrite dans l'étape d) du procédé de préparation, avant d'être mis en oeuvre dans le procédé d'hydrotraitement ou d'hydrocraquage selon l'invention. Cette étape de sulfuration, décrite dans l'étape d) génère la phase sulfure active de façon partielle, mais elle permet d'éviter la lixiviation des précurseurs métalliques au contact de la charge hydrocarbonée à traiter ou éventuellement au contact de la charge de sulfuration. Le catalyseur obtenu est mis en oeuvre dans une unité d'hydrotraitement ou d'hydrocraquage où il peut subir une sulfuration in situ, réalisée à l'aide de la charge à traiter en présence d'hydrogène et d'hydrogène sulfuré ($H_2S$) introduit tel quel ou issu de la décomposition d'un composé soufré organique choisi parmi le diméthyldisulfure (DMDS), le diméthylsulfure, le n-butylmercaptan et les composés polysulfures. Cette sulfuration est réalisée à une température comprise entre 200 et 600°C et de préférence comprise entre 300 et 400°C selon des procédés bien connus de l'Homme du métier.

## Les procédés d'hydrotraitement et d'hydrocraquage ainsi que les charges

Enfin, un autre objet de l'invention est l'utilisation du catalyseur selon l'invention dans des procédés d'hydrotraitement et d'hydrocraquage de coupes pétrolières.

Le catalyseur préparé avec le procédé selon l'invention peut avantageusement être utilisé dans tout procédé, connu de l'Homme du métier, nécessitant des réactions d'hydrogénation des coupes hydrocarbonées, et de préférence des coupes essences de craquage catalytique. Les procédés d'hydrotraitement et d'hydrocraquage selon l'invention peuvent avantageusement être mis en oeuvre dans tout type de réacteur opéré en lit fixe ou en lit mobile ou en lit bouillonnant. De préférence, ledit procédé d'hydrotraitement ou le dit procédé d'hydrocraquage est mis en oeuvre dans un réacteur opéré en lit fixe.

Les catalyseurs obtenus par le procédé de préparation selon l'invention sont avantageusement utilisés pour les réactions d'hydrotraitement de charges hydrocarbonées telles que les coupes pétrolières, les coupes issues du charbon ou les hydrocarbures produits à partir du gaz naturel et plus particulièrement nécessitant des réaction d'hydrogénation : citons les réactions d'hydrogénation des aromatiques, d'hydrodéazotation, d'hydrodésulfuration, d'hydrodémétallation ou d'hydrocraquage de charges hydrocarbonées .

Ces catalyseurs peuvent aussi avantageusement être utilisés lors du pré-traitement des charges de craquage catalytique ou l'hydrodésulfuration des résidus ou l'hydrodésulfuration poussée des gazoles (ULSD Ultra Low Sulfur Diesel).

Les charges employées dans les procédés d'hydrotraitement sont par exemple des essences, des gas-oils, des gas-oils sous vide, des résidus atmosphériques, des résidus sous vide, des distillats atmosphériques, des distillats sous vide, des fuels lourds, des huiles, des cires et des paraffines, des huiles usagées, des résidus ou des bruts désasphaltés, des charges provenant des procédés de conversions thermiques ou catalytiques, prises seules ou en mélanges. Les charges qui sont traitées, et en particulier celles citées ci-dessus, contiennent généralement des hétéroatomes tels que le soufre, l'oxygène et l'azote et, pour les charges lourdes, elles contiennent le plus souvent également des métaux.

Les conditions opératoires utilisées dans les procédés mettant en oeuvre les réactions d'hydrotraitement de charges hydrocarbonées décrites ci-dessus sont généralement les suivantes : la température est avantageusement comprise entre 180 et 450 °C, et de préférence entre 250 et 440 C, la pression est avantageusement comprise entre 0,5 et 30 MPa, et de préférence entre 1 et 18 MPa, la vitesse volumique horaire est avantageusement comprise entre 0,1 et 20 $h^{-1}$ et de préférence entre 0,2 et 5 $h^{-1}$, et le rapport hydrogène/charge exprimé en volume d'hydrogène, mesuré dans les conditions normales de température et pression, par volume de charge liquide est avantageusement compris entre 50 l/l à 2000 l/l.

Les charges employées dans les réactions d'hydrocraquage sont par exemple des LCO (Light Cycle Oil (gazoles légers issus d'une unité de craquage catalytique)), les distillats atmosphériques, les distillats sous vide par exemple gasoils issus de la distillation directe du brut ou d'unités de conversion telles que le FCC, le coker ou la viscoréduction, les charges provenant d'unités d'extraction d'aromatiques, des bases d'huile lubrifiante ou issues du déparaffinage au solvant des bases d'huile lubrifiante, les distillats provenant de procédés de désulfuration ou d'hydroconversion en lit fixe ou en lit bouillonnant, de résidus atmosphériques et/ou de résidus sous vide et/ou d'huiles désasphaltées, ou encore la charge peut être une huile désasphaltée ou comprendre des huiles végétales ou bien encore provenir de la conversion de charges issues de la biomasse. Ladite charge hydrocarbonée traitée selon le procédé d'hydrocraquage de l'invention peut encore être un mélange desdites charges précédemment citées. Les composés hydrocarbonés présents dans ladite charge sont des composés aromatiques, des composés oléfiniques, des composés naphténiques et/ou des composés paraffiniques.

Ladite charge hydrocarbonée comprend avantageusement des hétéroatomes. De manière préférée, lesdits hétéroatomes sont choisis parmi l'azote, le soufre et le mélange de ces deux éléments. Lorsque l'azote est présent dans ladite charge à traiter, la teneur en azote est supérieure ou égale à 500 ppm, de préférence elle est comprise entre 500 et 10000 ppm poids, de manière plus préférée entre 700 et 4000 ppm poids et de manière encore plus préférée entre 1000 et 4000 ppm. Lorsque le soufre est présent dans ladite charge à traiter, la teneur en soufre est comprise entre 0,01 et 5% poids, de manière préférée comprise entre 0,2 et 4% poids et de manière encore plus préférée entre 0,5 et 3 % poids.

Ladite charge hydrocarbonée peut éventuellement avantageusement contenir des métaux, en particulier du nickel et du vanadium. La teneur cumulée en nickel et vanadium de ladite charge hydrocarbonée, traitée selon le procédé d'hydrocraquage selon l'invention, est de préférence inférieure à 1 ppm poids. La teneur en asphaltènes de ladite charge hydrocarbonée est généralement inférieure à 3000 ppm, de manière préférée inférieure à 1000 ppm, de manière encore plus préférée inférieure à 200 ppm.

Le procédé d'hydrocraquage selon l'invention couvre les domaines de pression et de conversion allant de l'hydrocraquage doux à l'hydrocraquage haute pression. On entend par hydrocraquage doux, un hydrocraquage conduisant à des conversions modérées, généralement inférieures à 40%, et fonctionnant à basse pression, généralement entre 2 MPa et 10 MPa. Le procédé d'hydrocraquage selon l'invention est effectué en présence d'au moins un catalyseur d'hydrotraitement ou d'hydrocraquage selon l'invention. Le procédé d'hydrocraquage selon l'invention peut être réalisé en une ou deux étape(s), indépendamment de la pression à laquelle ledit procédé est mis en oeuvre. Il est effectué en présence d'un ou plusieurs catalyseur(s) obtenu(s) selon le procédé de préparation décrit ci-dessus, dans une ou plusieurs unité(s) réactionnelle(s) équipée(s) de un ou plusieurs réacteur(s).

Les conditions opératoires utilisées dans les procédés d'hydrocraquage selon l'invention peuvent être très variables en fonction de la nature de la charge, de la qualité des produits désirés et des installations dont dispose le raffineur. Conformément au procédé d'hydrocraquage selon l'invention, ledit catalyseur d'hydrocraquage est avantageusement mis en contact, en présence d'hydrogène, avec ladite charge hydrocarbonée à une température supérieure à 200°C, souvent comprise entre 250 et 480°C, avantageusement comprise entre 320 et 450°C, de préférence entre 330 et 435°C, sous une pression supérieure à 1 MPa, souvent comprise entre 2 et 25 MPa, de manière préférée entre 3 et 20 MPa, la vitesse spatiale (débit volumique de charge divisé par le volume du catalyseur) étant comprise entre 0,1 et 20$h^{-1}$ et de préférence entre 0,1 et 6$h^{-1}$, de manière encore plus préférée entre 0,2 et 3$h^{-1}$, et la quantité d'hydrogène introduite est telle que le rapport volumique litre d'hydrogène / litre d'hydrocarbure soit compris entre 80 et 5000l/l et le plus souvent entre 100 et 2000 l/l.

Ces conditions opératoires utilisées dans le procédé d'hydrocraquage selon l'invention permettent généralement d'atteindre des conversions par passe, en produits ayant des points d'ébullition d'au plus 370°C et avantageusement d'au plus 340°C, supérieures à 15% et de manière encore plus préférée comprises entre 20 et 95%.

## Exemples

Les exemples qui suivent sont présentés à titre illustratif, ils démontrent le gain d'activité important sur les catalyseurs préparés selon le procédé selon l'invention par rapport aux catalyseurs de l'art antérieur et précisent l'invention sans toutefois en limiter la portée.

### Exemple 1 : Catalyseur NiMo supporté sur alumine, avec une densité surfacique de 3 Mo/nm$^2$ et Ni/Mo=0,3 (at/at). (conforme)

Le molybdène est imprégné à sec en milieu strictement non aqueux sur un support de type $\gamma$-alumine commerciale synthétisée par calcination d'un gel de sels d'aluminium (287 m$^2$/g). Le support est préalablement calciné à 300°C sous air durant 6 heures à pression atmosphérique. Il est ensuite chauffé à 300°C durant 14 heures sous vide secondaire (10$^{-5}$ mbar) avant d'être stocké en milieu inerte, en boite à gants. Le précurseur du molybdène est le pentaéthoxyde de molybdène Mo(OC$_2$H$_5$)$_5$. Du dichlorométhane sec et dégazé est utilisé comme solvant. 1,96 mL de solution d'imprégnation, préparée à partir de 1,18 g de précurseur, sont imprégnés sur 2,58 g de support sec. La quantité de molybdène est ajustée de façon à obtenir environ 3 Mo/nm$^2$. Après une maturation de 15 heures, les extrudés sont séchés sous vide (10$^{-5}$ mbar) durant 2 heures, à température ambiante. Ce catalyseur non sulfuré est défini par la notation usuelle Mo / Al$_2$O$_3$.

Une solution de nickel bis-acétylacétonate Ni(acac)$_2$ est ensuite imprégnée sur ce catalyseur. Du toluène sec et dégazé est utilisé comme solvant. Le précurseur de nickel est préalablement solubilisé à chaud dans le toluène, puis 2,45 mL d'une solution contenant 0,41 g de précurseur est imprégnée sur environ 3,76 g de Mo / Al$_2$O$_3$. Après une maturation de 15 heures, les extrudés sont séchés sous vide (10$^{-5}$ mbar) durant 3 heures, à température ambiante. Sur ce catalyseur non sulfuré NiMo / Al$_2$O$_3$, les teneurs en molybdène et en nickel sont respectivement de 11,60 %$_{pds.}$ et 2,21 %$_{pds.}$ ce qui correspond à une densité surfacique réelle de 3,0 Mo/nm$^2$ et un rapport atomique Ni/Mo = 0,30. Ce catalyseur C1 est conforme à l'invention.

**Exemple 2 : Catalyseur NiMo supporté sur alumine, avec une densité surfacique de 3 Mo/nm$^2$ et Ni/Mo=0,3 (at/at) (non conforme)**

Le molybdène et le nickel sont co-imprégnés à sec en milieu aqueux sur un support de type $\gamma$-alumine commerciale synthétisée par calcination d'un gel de sels d'aluminium (289 m$^2$/g). Le précurseur du molybdène est l'heptamolybdate d'ammonium $(NH_4)_6Mo_7O_{24}$, $xH_2O$. Le précurseur du nickel est le nitrate de nickel $Ni(NO_3)_2$, $xH_2O$. Les quantités de précurseurs sont ajustées de façon à obtenir 3 Mo/nm$^2$ et Ni/Mo=0,30 (at/at). Après une maturation de 15 heures, les extrudés sont séchés à 120°C durant 15 heures. Ils sont ensuite calcinés à 450°C sous flux d'air, durant 2 heures. Sur ce catalyseur non sulfuré NiMo / $Al_2O_3$, les teneurs en molybdène et en nickel sont respectivement de 11,75 %$_{pds.}$ et 2,12 %$_{pds.}$ ce qui correspond à une densité surfacique réelle de 3,0 Mo/nm$^2$ et un rapport atomique Ni/Mo = 0,29. Ce catalyseur H1 est non-conforme.

**Exemple 3 : Test en hydrogénation du toluène (molécule modèle aromatique) en présence d'aniline.**

Le test d'hydrogénation du toluène en présence d'aniline a pour but l'évaluation de l'activité hydrogénante des catalyseurs sulfurés supportés ou massiques, en présence d'$H_2S$ et sous pression d'hydrogène. L'isomérisation qui caractérise la fonction acide du catalyseur est inhibée par la présence de l'aniline, à faible température et/ou par la présence de $NH_3$ (issu de la décomposition de l'aniline) à plus forte température. L'aniline et/ou $NH_3$ vont réagir via une réaction acide base avec les sites acides du support. Les réactions d'isomérisation caractéristiques de l'acidité du support sont alors inexistantes.

Nous avons pris soin de réaliser la comparaison des catalyseurs sur la même unité de test catalytique de façon à ne pas fausser les comparaisons par l'utilisation d'outils de test catalytique différents pouvant engendrer des résultats décalés.

Le test catalytique se déroule en phase gaz, dans un réacteur en lit fixe traversé. Le test se décompose en deux phases distinctes, la sulfuration et le test catalytique. Le test est réalisé à 60 bar.

Les catalyseurs ont été préalablement sulfurés, ex-situ, sous phase gaz (mélange ($H_2S/H_2$) dans lequel la quantité d'$H_2S$ est de 15% volumique) à une température de 350°C pendant 2h.

Phase d'activation :

Les catalyseurs subissent une montée en température, sous charge de test, dans un réacteur tubulaire à lit fixe traversé d'une unité pilote de type Flowrence (constructeur Avantium), les fluides circulant de haut en bas. Les mesures d'activité hydrogénante sont effectuées immédiatement après atteinte de la température de test.

Test catalytique :

La charge de test est composée de diméthyldisulfure (DMDS), de toluène, de cyclohexane et d'aniline.

On mesure les activités catalytiques stabilisées de volumes égaux de catalyseurs (450 $\mu$L) et à une température de 350 °C.

Les conditions opératoires du test sont les suivantes (en considérant une vaporisation totale, et la loi des gaz parfaits) :

pour Ptot = 60 bar et T = 350°C :

PpH$_2$ = 36,62 bar
PpNH$_3$ = 0,09 bar
PpH$_2$S = 2,16 bar
Pptoluène = 3,75 bar
Ppcyclohexane = 15,22 bar
VVH = 4 l/l/h lors de la phase d'activation, et VVH = 2 l/l/h et $H_2$/charge = 450 l/l lors de la phase de test.

Des prélèvements des effluents sont analysés par chromatographie en phase gazeuse. Les performances catalytiques des catalyseurs sont exprimées à l'aide de l'activité hydrogénante qui correspond, en suivant une loi cinétique d'ordre 1, à :

$$AH_{ordre.1} = \ln \frac{100}{(100 - \%HYD_{tolu\grave{e}ne})}$$

$\%HYD_{tolu\grave{e}ne}$ correspond au pourcentage de toluène hydrogéné.

Les performances catalytiques sont rassemblées dans le tableau 1.

**Tableau 1 : Activité hydrogénante relative des catalyseurs C1 et H1.**

| Elles sont exprimées en activité relative, en posant que celle du catalyseur H1 est égale à 100. | |
|---|---|
| Catalyseur | Activité hydrogénante relative à H1 |
| C1 (conforme) | 145 |
| H1 (non conforme) | 100 |

Le tableau 1 montre le gain de pouvoir hydrogénant important obtenu sur le catalyseur revendiqué selon l'invention, préparé sur alumine (C1). Le catalyseur C1, préparé à partir du précurseur de molybdène Mo(OEt)$_5$ selon l'invention, est plus actif en hydrogénation que le catalyseur qui est son homologue de formulation, mais préparé par voie classique, utilisant un sel d'hétéropolyanion (H1).

**Revendications**

1. Procédé de préparation d'un catalyseur renfermant au moins un support, éventuellement au moins un métal du groupe VIII de la classification périodique des éléments, et au moins du molybdène, ledit procédé étant **caractérisé en ce que** :

   - on introduit sur le support le molybdène dans un solvant organique A sous la forme d'au moins un composé précurseur mononucléaire à base de Mo, sous sa forme monomérique ou dimérique, présentant au moins une liaison Mo=O ou Mo-OR ou au moins une liaison Mo=S ou Mo-SR où [R = C$_x$H$_y$ où x ≥ 1 et (x-1) ≤ y ≤ (2x+1) ou R = Si(OR')$_3$ ou R = Si(R')$_3$ où R' = C$_{x'}$H$_{y'}$ où x' ≥ 1 et (x'-1) ≤ y' ≤ (2x'+1)],
   - et on effectue un traitement thermique du support ainsi imprégné par séchage à une température inférieure à 200 °C.

2. Procédé de préparation selon la revendication 1 dans lequel ledit séchage est effectué sous atmosphère anhydre ou sous vide primaire ou sous vide secondaire ou sous flux de gaz inerte.

3. Procédé selon la revendication 2 dans lequel ledit séchage est effectué sous vide et à température ambiante.

4. Procédé selon la revendication 1 à 3 dans lequel le métal du groupe VIII est choisi parmi le cobalt, le fer ou le nickel

5. Procédé selon la revendication 4 tel que le métal du groupe VIII est le nickel.

6. Procédé selon l'une des revendications 1 à 5 tel que le précurseur du molybdène est un précurseur mononucléaire à base de Mo, utilisé sous sa forme monomérique ou dimérique, de formule Mo(=O)$_n$(=S)$_{n'}$(OR)$_a$(SR')$_b$(L1)$_c$(L2)$_d$(L3)$_e$(L4)$_f$(L5)$_g$
   où R = C$_x$H$_y$ où x ≥ 1 et (x-1) ≤ y ≤ (2x+1) ou R = Si(OR'')$_3$ ou R = Si(R'')$_3$ où R'' = C$_{x''}$H$_{y''}$ où [x'' ≥ 1 et (x''-1) ≤ y'' ≤ (2x''+1)],
   où R' = C$_{x'}$Hy' où x' ≥ 1 et (x'-1) ≤ y' ≤ (2x'+1) ou R' = Si(OR''')$_3$ ou R' = Si(R''')$_3$ où R''' = C$_{x'''}$H$_{y'''}$ où [x''' ≥ 1 et (x'''-1) ≤ y''' ≤ (2x'''+1)], où 0 ≤ n+n' ≤ 2 et 0 ≤ n ≤ 2 et 0 ≤ n' ≤ 2,
   où, si n = n' = 0, alors (a#0 ou b#0) et [(a+b+c+d+e+f+g = 6 et 0 ≤ a ≤ 6, 0 ≤ b ≤ 6, 0 ≤ c ≤ 6, 0 ≤ d ≤ 6, 0 ≤ e ≤ 6, 0 ≤ f ≤ 6, 0 ≤ g ≤ 6, ou (a+b+c+d+e+f+g = 5 et 0 ≤ a ≤ 5, 0 ≤ b ≤ 5, 0 ≤ c ≤ 5, 0 ≤ d ≤ 5, 0 ≤ e ≤ 5, 0 ≤ f ≤ 5, 0 ≤ g ≤ 5),ou (a+b+c+d+e+f+g = 4 et 0 ≤ a ≤ 4, 0 ≤ b ≤ 4, 0 ≤ c ≤ 4, 0 ≤ d ≤ 4, 0 ≤ e ≤ 4, 0 ≤ f ≤ 4, 0 ≤ g ≤ 4)]
   où, si [(n=1 et n' = 0) ou (n' = 1 et n = 0)], alors [a+b+c+d+e+f+g = 4 et 0 ≤ a ≤ 4, 0 ≤ b ≤ 4, 0 ≤ c ≤ 4, 0 ≤ d ≤ 4, 0 ≤ e ≤ 4, 0 ≤ f ≤ 4, 0 ≤ g ≤ 4)] ou [(a+b+c+d+e+f+g = 3 et 0 ≤ a ≤ 3, 0 ≤ b ≤ 3, 0 ≤ c ≤ 3, 0 ≤ d ≤ 3, 0 ≤ e ≤ 3, 0 ≤ f ≤ 3, 0 ≤ g ≤ 3)]
   où, si [n+n'=2 et 0 ≤ n ≤ 2 et 0 ≤ n' ≤ 2], alors (a+b+c+d+e+f+g = 2 et 0 ≤ a ≤ 2, 0 ≤ b ≤ 2, 0 ≤ c ≤ 2, 0 ≤ d ≤ 2, 0 ≤ e ≤ 2, 0 ≤ f ≤ 2, 0 ≤ g ≤ 2),

avec (L1), (L2), (L3), (L4) et (L5) choisis parmi les ligands de type THF, dimethyl ether, dimethylsulfure, P(CH$_3$)$_3$, allyl, aryl, halogénés, amine, acétate, acétylacétonate, halogénure, hydroxyde, -SH.

7. Procédé selon la revendication 6 dans lequel le précurseur de molybdène est choisi parmi Mo(OEt)$_5$, Mo(OEt)$_6$, Mo(=O)(OEt)$_4$, Mo(=S)(OEt)$_4$, Mo(=S)(SEt)$_4$, Mo(=O)$_2$(OEt)$_2$, Mo(OC$_6$H$_5$)$_6$, Mo(SEt)$_5$, Mo(SEt)$_6$, Mo(OEt)(SEt)$_4$, Mo(OEt)$_2$(SEt)$_3$, Mo(OEt)$_3$(SEt)$_2$, Mo(OEt)$_4$(SEt), Mo(=O)(OEt)$_3$(acac) avec Et = CH$_2$CH$_3$ (groupement éthyle) et acac = (CH$_3$COCHCOCH$_3$)-(acétylacétonate) sous leur forme monomérique ou dimérique.

8. Procédé de préparation d'un catalyseur selon l'une des revendications 1 à 7 tel que le molybdène, et le ou les métaux du groupe VIII éventuels sont introduits simultanément ou successivement.

9. Procédé de préparation d'un catalyseur selon l'une des revendications 1 à 8 comprenant au moins une étape finale de sulfuration en phase gaz, in situ et/ou ex situ.

10. Procédé de préparation d'un catalyseur selon l'une des revendications précédentes qui comprend au moins les étapes suivantes :

a) imprégnation par mise en contact d'une solution S comprenant le solvant organique A et au moins ledit précurseur mononucléaire à base de molybdène, sous sa forme monomérique ou dimérique, présentant au moins une liaison Mo=O ou Mo-OR ou au moins une liaison Mo=S ou Mo-SR où [R = C$_x$H$_y$ où x ≥ 1 et (x-1) ≤ y ≤ (2x+1) ou R = Si(OR')$_3$ ou R = Si(R')$_3$ où R' = C$_{x'}$H$_{y'}$ où x' ≥ 1 et (x'-1) ≤ y' ≤ (2x'+1)], avec un support minéral poreux, préalablement calciné sous vide primaire ou sous vide secondaire ou sous flux de gaz inerte ;
b) maturation sous atmosphère anhydre ;
c) séchage du support imprégné à une température inférieure à 200°C sous atmosphère anhydre ou sous vide primaire ou sous vide secondaire ou sous flux de gaz inerte.
d) sulfuration ex-situ sous un mélange H$_2$S/H$_2$ ou H$_2$S/N$_2$ contenant au moins 5% volumique d'H$_2$S dans le mélange à une température égale ou supérieure à la température ambiante.

11. Procédé de préparation d'un catalyseur selon la revendication 10 dans lequel le métal du groupe VIII éventuel est introduit à l'étape a) dans la même solution S que le précurseur de molybdène, ou après le séchage c) dans une étape de post-imprégnation a2) à l'aide d'une solution utilisant un solvant organique B, ou après l'étape d) de sulfuration, dans une étape de post-imprégnation a3) à l'aide d'une solution aqueuse ou organique.

12. Catalyseur susceptible d'être préparé selon l'une des revendications 1 à 11.

13. Catalyseur selon la revendication 12 comprenant une teneur en molybdène comprise entre 4 et 30 % poids et une teneur en métal(aux) du groupe VIII comprise entre 0,1 à 8% poids par rapport au poids total de catalyseur.

14. Utilisation d'un catalyseur selon la revendication 12 ou 13 ou préparé selon l'une des revendications 1 à 11 dans les réactions d'hydrogénation de charges hydrocarbonées.

15. Utilisation selon la revendication 14 pour l'hydrotraitement ou l'hydrocraquage.

Europäisches
Patentamt

European
Patent Office

Office européen
des brevets

**RAPPORT DE RECHERCHE EUROPEENNE**

Numéro de la demande

EP 14 30 5437

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 94/21375 A1 (BORESKOVA INST KATALIZA SIBIR [RU]; STARTSEV ANATOLY NIKOLAEVICH [RU];) 29 septembre 1994 (1994-09-29) * abrégé * * page 3, ligne 16-21 * * page 15, ligne 26-32 * * page 17, ligne 23-24 * * exemples 3,11,22 * ----- | 1,2, 4-10, 12-15 | INV. B01J23/883 B01J23/28 B01J37/02 B01J37/20 C10G45/08 C10G47/12 ADD. |
| X,D | KALUZA L ET AL: "Hydrotreating catalysts supported on organized mesoporous alumina: Optimization of Mo deposition and promotional effects of Co and Ni", APPLIED CATALYSIS A: GENERAL, ELSEVIER SCIENCE, AMSTERDAM, NL, vol. 351, no. 1, 15 décembre 2008 (2008-12-15), pages 93-101, XP025609038, ISSN: 0926-860X, DOI: 10.1016/J.APCATA.2008.09.002 [extrait le 2008-09-10] * pages 93-95; tableaux 1,2 * * page 101 * ----- | 1,2,4-6, 8-15 | B01J31/22 B01J31/02 |
| X,D A | EP 0 601 722 A1 (SUMITOMO METAL MINING CO [JP]) 15 juin 1994 (1994-06-15) * page 2, ligne 1-16 * * page 3, ligne 25-29 * * page 3, ligne 43-49 * * page 6, ligne 1-44 * * page 7, ligne 45-50 * * revendications 1,8-11; exemples 2-36; tableau 55; composés GCE,GCF * ----- -/-- | 12-15 1-11 | DOMAINES TECHNIQUES RECHERCHES (IPC) B01J C10G |

Le présent rapport a été établi pour toutes les revendications

| Lieu de la recherche | Date d'achèvement de la recherche | Examinateur |
|---|---|---|
| Munich | 29 août 2014 | Goebel, Matthias |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## EP 2 799 131 A1

## RAPPORT DE RECHERCHE EUROPEENNE

Numéro de la demande

EP 14 30 5437

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | EP 0 451 640 A2 (COSMO SOGO KENKYUSHO KK [JP]; COSMO OIL CO LTD [JP]) 16 octobre 1991 (1991-10-16) * page 4, ligne 51-54; revendication 1; exemples 1-4; tableau 1 * ----- | 1,4,6-8, 12-15 | |
| X | DONGREN WANG ET AL: "Polymer-Supported Chiral Schrock Catalysts Immobilizedvia the Arylimido Ligand", ADVANCED SYNTHESIS & CATALYSIS, vol. 348, no. 12-13, 1 août 2006 (2006-08-01), pages 1567-1579, XP055112960, ISSN: 1615-4150, DOI: 10.1002/adsc.200606030 * abrégé * * Schéma 2; composés 22,24,25,26,27,28 * * page 1577 - page 1578 * ----- | 1-3,6,12 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Munich | 29 août 2014 | Goebel, Matthias |

EPO FORM 1503 03.82 (P04C02)

EP 2 799 131 A1

**ANNEXE AU RAPPORT DE RECHERCHE EUROPEENNE**
**RELATIF A LA DEMANDE DE BREVET EUROPEEN NO.**          EP 14 30 5437

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-08-2014

| Document brevet cité au rapport de recherche | | Date de publication | Membre(s) de la famille de brevet(s) | | Date de publication |
|---|---|---|---|---|---|
| WO 9421375 | A1 | 29-09-1994 | RU | 2052285 C1 | 20-01-1996 |
| | | | WO | 9421375 A1 | 29-09-1994 |
| EP 0601722 | A1 | 15-06-1994 | CA | 2103337 A1 | 19-05-1994 |
| | | | DE | 69321203 D1 | 29-10-1998 |
| | | | DE | 69321203 T2 | 02-06-1999 |
| | | | DK | 0601722 T3 | 14-06-1999 |
| | | | EP | 0601722 A1 | 15-06-1994 |
| | | | JP | 2900771 B2 | 02-06-1999 |
| | | | JP | H06226108 A | 16-08-1994 |
| | | | US | 5468709 A | 21-11-1995 |
| EP 0451640 | A2 | 16-10-1991 | DE | 69100579 D1 | 09-12-1993 |
| | | | DE | 69100579 T2 | 14-04-1994 |
| | | | EP | 0451640 A2 | 16-10-1991 |
| | | | JP | H0813330 B2 | 14-02-1996 |
| | | | JP | H03284353 A | 16-12-1991 |
| | | | US | 5137859 A | 11-08-1992 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2547380 A
- FR 2749778
- FR 2764211
- FR 2315721
- FR 2969647
- FR 2969645
- WO 9641848 A
- WO 0176741 A
- US 4012340 A
- US 3954673 A

- EP 601722 A
- EP 0466568 A
- EP 1046424 A
- FR 2880823
- FR 2953740
- JP 7136523 A
- US 2963360 A
- EP 0178711 A
- US 5137859 A

**Littérature non-brevet citée dans la description**

- **H. TOULHOUAT ; P. RAYBAUD.** *Catalysis by transition metal sulphides, From Molecular Theory to Industrial Application,* 2013
- **J. SCHERZER ; A. J. GRUIA.** Hydrocracking Science and Technology. Marcel Dekker Inc, 1996
- **YERMAKOV et al.** *Journal of Molecular Catalysis,* 1981, 205-214
- **YERMAKOV.** *Journal of molecular catalysis,* 1983, vol. 21, 35-55

- **YERMAKOV et al.** *Applied Catalysis,* 1984, vol. 11, 1-13
- **HALBERT et al.** *Journal of Catalysis,* 1991, vol. 130, 116-129
- **KALUZA et al.** *Applied Catalysis A : General,* 2008, vol. 351, 93-101
- **BAERLOCHER, L. B. ; L.MCCUSKER ; D. H. OLSON.** Atlas of zéolite framework types. 2007